# EUROPEAN PATENT SPECIFICATION

(11) **EP 1 146 028 B1**
(45) Date of publication and mention of the grant of the patent: **19.02.2003**
(21) Application number: 01302930.1
(22) Date of filing: 28.03.2001
(51) Int. Cl.: C07B 37/10, C07C 13/04, C07C 1/30, C07C 1/32

(54) **A method to prepare cyclopropenes**
Verfahren zur Herstellung von Cyclopropenen
Procédé pour la préparation de cyclopropènes

(30) Priority: 11.04.2000 US 196536
(43) Date of publication of application: 17.10.2001
(73) Proprietor: ROHM AND HAAS COMPANY, Philadelphia, Pennsylvania 19106-2399 (US)
(72) Inventor: Jacobson, Richard Martin, Chalfont, Pennsylvania 18914 (US)
(74) Representative: Kent, Venetia Katherine

(56) References cited:
- DE-A- 4 333 491
- US-A- 5 723 714

## Description

The present invention relates to a method to prepare cyclopropenes.

Cyclopropenes have a variety of uses in the chemical industry. One recent discovery is the ability of 1-methylcyclopropene, and related analogs, to inhibit the action of ethylene on plants (see U. S. Patent No. 5,518,988). Several syntheses for cyclopropene and its simple derivatives have been reported. The most widely practiced process is the sodium amide induced α-elimination of an allylic chloride (see F. Fisher and D. Applequist, *J*. *Org. Chem.,* 30, 2089 (1965)). Unfortunately, this method suffers from relatively low yields, difficulty in purifying the final product, and the use of excess sodium amide. An improved synthesis of 1-methylcyclopropene incorporates the use of freshly prepared halide free phenyllithium as the base replacing sodium amide. This method provides overall yields in the 60 to 80 percent range (see R. Magid, et. al., *J*. *Org. Chem.,* 36, 1320 (1971)). Similar synthesis methods are disclosed in U. S. Patent No. 6,017,849.

Although the reported reactions work reasonably well, a major problem often encountered is the presence of unwanted isomers such as methylenecyclopropane in the final product mixture. Methods which improve the isomer ratio (that is, increased 1-methylcyclopropene compared to the methylenecyclopropane often do so at the expense of overall yield of product (see Koster, et. al., *Liebigs Ann. Chem*., 1219 (1973)). Because of these problems, there is still a need for a method to prepare 1-methylcyclopropene in high yield and in high isomer purity. I have discovered that when a catalytic amount of certain weaker bases is added to the reaction mixture of a substituted or unsubstituted allyl halide and a strong base in an inert solvent, cyclopropenes, with greatly reduced unwanted isomer content, are produced in high yield.

US 5 723 714 teaches an industrial process for the preparation of methylene cyclopropane from 3-halogen-2-methyl-1-propene with an alkali metal-bis-(trialkylsilyl)-amide, optionally in the presence of an alkali metal alcoholate. Minor amounts of 1-methyl cyclopropane are formed.

The present invention, therefore, is a method to prepare cyclopropenes, comprising combining an allyl compound of formula I: 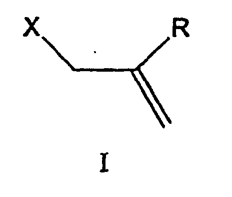 wherein:
- X: is a leaving group; and
- R: is hydrogen or a substituted or unsubstituted alkyl, alkenyl, alkynyl, cycloalkyl, cycloalkylalkyl, phenyl, or naphthyl group; wherein the substituents are independently halogen, alkoxy, or substituted or unsubstituted phenoxy;
with a non-nucleophilic strong base with a pKₐ greater than 35 in an inert solvent in the presence of a catalytic amount of a weaker, non-nucleophilic base.

As used herein, the term "alkyl" means both straight and branched chain (C₁-C₂₀) radicals which include, for example, methyl, ethyl, n-propyl, isopropyl, 1-ethylpropyl, n-butyl, tert-butyl, isobutyl, 2,2-dimethylpropyl, pentyl, octyl, and decyl. The terms "alkenyl" and "alkynyl" mean (C₃-C₂₀) alkenyl and alkynyl groups such as, for example, 2-propenyl, 2-butenyl, 3-butenyl, 2-methyl-2-propenyl, and 2-propynyl. The term "cycloalkylalkyl" means a (C₁-C₁₅) alkyl group substituted with a (C₃-C₆) cycloalkyl group such as, for example cyclopropylmethyl, cyclopropylethyl, cyclobutylmethyl, and cyclopentylethyl. The term "haloalkyl" means an alkyl radical wherein one or more of the hydrogen atoms have been replaced by a halogen atom. The term "halogen" means fluorine, chlorine, bromine, and iodine. The term "strong base" means a non-nucleophilic base with a pKₐ greater than 35. The term "weaker base" means a non-nucleophilic base with a pKₐ of from 26 to 35, or the conjugate acid thereof. The term "inert solvent" means a solvent which does not react with the strong base, the weaker base, the allyl halide, or the resulting cyclopropene.

As used herein, all percentages are percent by weight, unless otherwise specified and are inclusive and combinable. All ratios are by weight and all ratio ranges are inclusive and combinable. All molar ranges are inclusive and combinable.

Preferably, X is a leaving group selected from halogen, alkyl or aryl sulfonyloxy, alkyl or aryl sulfate, and alkoxy. More preferably, X is chloro, bromo, iodo, benzenesulfonyloxy, p-toluenesulfonyloxy, methanesulfonyloxy, or *t*-butoxy. Even more preferably, X is chloro, bromo, or benzenesulfonyloxy. Most preferably, X is chloro or bromo.

Preferably, R is (C₁-C₁₀) alkyl. More preferably, R is (C₁-C₈) alkyl. Most preferably, R is methyl.

Preferably, the inert solvent is an aliphatic or aromatic hydrocarbon such as, for example, mineral oil, benzene, toluene, or xylene; an ether such as, for example, diethyl ether, tetrahydrofuran, or dioxane, a halogenated hydrocarbon such as, for example perhaloalkanes or methylene chloride, liquid ammonia, or lower alkyl amine or lower dialkyl amine such as, for example, methylamine or dimethylamine. More preferably, the solvent is an aliphatic or aromatic hydrocarbon. Most preferably, the solvent is mineral oil. The solvent may be a mixture of more than one inert solvent.

Preferably, the strong base is an alkali metal salt of an amine or an organometallic base. More preferably, the strong base is sodium, potassium, or lithium amide or phenylithium. Most preferably, the strong base is sodium amide. The strong base may be a mixture of more than one strong base. The amount of strong base used in the method will vary depending upon the weaker base used, the inert solvent, and the temperature at which the reaction is conducted. Preferably, the amount of strong base used is from 0.1 to 20 moles per mole of allyl compound of formula I. More preferably the amount of strong base used is from 0.5 to 2 moles per mole of allyl compound of formula I. Most preferably, the amount of strong base used is from 0.7 to 1.4 moles per mole of allyl compound of formula I.

Preferably, the weaker base is soluble in the inert solvent. More preferably, the weaker base is a silyl amine, a disilazane, their cyclic analogs, mixed cyclic silazane/ether analogs, or metal salts thereof. Even more preferably, the weaker base is a silyl amine or a disilazane. Still more preferably, the weaker base is a dialkyl- or trialkyl, diaryl- or triaryl, or mixed alkyl/aryl silyl amine; a tetraalkyl-, pentaalkyl- or hexaalkyl, tetraaryl-, pentaaryl-, or hexaaryl, or mixed alkyl/aryl disilazane; or their cyclic analogs. Still more preferably, the weaker base is 1,1,1-triphenylsilylamine, tri-n-hexylsilylamine, 1,1,1,3,3,3-hexamethyldisilazane, 1,1,3,3-tetramethyldisilazane, 2,2,4,4,6,6-hexamethylcyclotrisilazane, octamethylcyclotetrasilazane, hexaethyldisilazane, 1,3-di-n-octyltetramethyldisilazane, or 2,2,5,5-tetramethyl-2,5-disila-1-azacyclopentane. Most preferably, the weaker base is hexamethyldisilazane. The weaker base can be a mixture of more than one weaker base. The amount of weaker base used in the method of this invention will vary depending upon the strong base used, the inert solvent used, and the temperature at which the reaction is conducted. Preferably, the amount of weaker base used is from 0.001 to 0.95 moles per mole of strong base used. More preferably, the amount of weaker base used is from 0.02 to 0.4 moles per mole of strong base. Most preferably, the amount of weaker base used is from 0.02 to 0.2 moles per mole of strong base.

The order of addition of the reactants to the solvent is not critical. However, it is preferred that the allyl compound of formula I is added last to a mixture of the other components. Most preferably, the allyl compound is added slowly to a mixture of the other components.

The temperature at which the method of this invention is carried out is not critical. However, because cyclopropenes are reactive compounds, care must be taken to ensure either a) that the temperature is kept below that at which decomposition or side reactions occur, or b) the cyclopropene produced must be removed from the reaction mixture as it is being produced, or a combination of a) and b). Preferably, the cyclopropene produced will be distilled from the reaction mixture and collected in a cooled receiver as it is produced. For lower boiling cyclopropenes, the temperature is preferably greater than or equal to the boiling point of the cyclopropene and the cyclopropene is distilled from the reaction mixture as it is being produced. For higher boiling cyclopropenes, the temperature is preferably less than the decomposition temperature of the cyclopropene.

Preferably, the reaction mixture is stirred or otherwise agitated and/or sparged or purged with an inert gas during the reaction. Preferably the inert gas is nitrogen. More preferably, and particularly in the case of lower boiling cyclopropenes, the agitation rate is sufficiently high to ensure that the cyclopropene distills from the reaction mixture as soon as possible after being formed. Fast removal of the cyclopropene from the reaction mixture reduces production of side products such as alkylidenecyclopropanes and teleomers.

The pressure at which the reaction is conducted is also not critical. Ambient pressure is preferred. However, pressure or vacuum may be utilized to affect the relative boiling points of the cyclopropene produced and the inert solvent to aid in separation of the cyclopropene from the reaction mixture.

The method of this invention is illustrated by the following examples and comparative examples. In the following examples, all percentages and parts are by weight.

### Preparation of 1-methylcyclopropene with catalysis:

Into a 1000 ml 4 necked round bottomed flask equipped with an internal thermometer, overhead stirring, a pressure equalizing addition funnel and a condenser was added 109 g (2.79 moles) of commercial sodium amide and 110 ml of light mineral oil. The flask was heated, via an external bath, to 45 °C internal temperature whereupon 4.2 g (0.03 moles) of hexamethyldisilazane was added. Over the course of 50 minutes, 202 g (2.23 moles) of 3-chloro-2-methylpropene was slowly added. The gas that was evolved was ducted through the condenser and then scrubbed with water and finally condensed in a dry ice cooled trap. Yield was 39.3 g of 1-methylcyclopropene contaminated with 1.5 percent of methylenecyclopropane and 8 percent of 3-chloro-2-methylpropene starting material. A simple distillation of the product gave a final product containing less than 0.1 percent 3-chloro-2-methylpropene.

### Preparation of 1-methylcyclopropene in the absence of catalyst:

Into a 1000 ml 4 necked round bottomed flask equipped with an internal thermometer, overhead stirring, a pressure equalizing addition funnel and a condenser was added 33.4 g (0.856 moles) of commercial sodium amide and 100 ml of light mineral oil. The flask was heated, via an external bath, to 45 °C internal temperature. Over the course of 90 minutes, 36.7 g (0.405 moles) of 3-chloro-2-methylpropene was slowly added. No gas was evolved indicating either a) no 1-methylcyclopropene was produced or b) any 1-methylcyclopropene which was produced decomposed or engaged in further reaction(s).

## Claims

1. A process to prepare a cyclopropene, comprising combining an allyl compound of the formula: 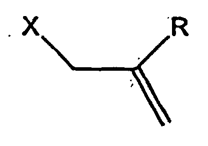 wherein:
X is a leaving group; and
R is hydrogen or a substituted or unsubstituted alkyl, alkenyl, alkynyl, cycloalkyl, cycloalkylalkyl, phenyl or naphthyl group wherein the substituents are independently halogen, alkoxy, or substituted or unsubstituted phenoxy;
with a non-nucleophilic strong base with a pKₐ greater than 35 in an inert solvent in the presence of a catalytic amount of a non-nucleophilic, weaker base.

2. The process of claim 1, wherein X is halogen, alkyl or aryl sulfonyloxy, alkyl or aryl sulfate, and alkoxy.

3. The process of claim 2, wherein X is chloro, bromo, iodo, benzenesulfonyloxy, p-toluenesulfonyloxy, methanesulfonyloxy, or *t*-butoxy.

4. The process of any preceding claim, wherein R is (C₁-C₁₀) alkyl.

5. The process of any preceding claim, wherein the strong base is an alkali metal salt of an amine or an organometallic base.

6. The process of any preceding claim, wherein the weaker base is a silyl amine or a disilazane.

7. The process of any preceding claim, wherein the inert solvent is an aliphatic or aromatic hydrocarbon; an ether, a halogenated hydrocarbon, liquid ammonia, methylamine, or dimethylamine

8. The process of any preceding claim, wherein the method further comprises removing the cyclopropene from the reaction mixture by distillation.

9. The process of any preceding claim, wherein X is chloro or bromo, R is (C₁-C₁₀) alkyl, the strong base is sodium amide, the weaker base is hexamethyldisilazane, and the inert solvent is mineral oil.

10. The process of claim 9, wherein R is methyl.

## Patentansprüche

1. Verfahren zur Herstellung eines Cyclopropens, umfassend das Kombinieren einer Allylverbindung der Formel: 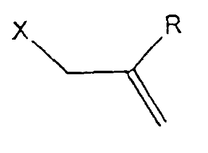 worin:
X eine Abgangsgruppe ist; und
R ein Wasserstoffatom oder ein substituierter oder unsubstituierter Alkyl-, Alkenyl-, Alkinyl-, Cycloalkyl-, Cycloalkyl-Alkylrest, eine Phenyl- oder Naphthylgruppe ist, wobei die Substituenten unabhängig Halogenatome, Alkoxyreste oder substituierte oder unsubstituierte Phenoxygruppen sind;
mit einer nicht nukleophilen, starken Base mit einem pKa von größer als 35 in einem inerten Lösungsmittel in der Gegenwart einer katalytischen Menge einer nicht nukleophilen, schwächeren Base.

2. Verfahren nach Anspruch 1, wobei X ein Halogenatom, ein Alkyl- oder Arylsulfonyloxyrest, ein Alkyl- oder Arylsulfatrest und ein Alkoxylrest ist.

3. Verfahren nach Anspruch 2, wobei X ein Chlor-, Brom-, lodatom, eine Benzolsulfonyloxy-, p-Toluolsulfonyloxy-, Methansulfonyloxy- oder *t*-Butoxygruppe ist.

4. Verfahren nach einem vorhergehenden Anspruch, wobei R ein (C₁-C₁₀) Alkylrest ist.

5. Verfahren nach einem vorhergehenden Anspruch, wobei die starke Base ein Alkalimetallsalz eines Amins oder eine metallorganische Base ist.

6. Verfahren nach einem vorhergehenden Anspruch, wobei die schwächere Base ein Silylamin oder ein Disilazan ist.

7. Verfahren nach einem vorhergehenden Anspruch, wobei das inerte Lösungsmittel ein aliphatischer oder aromatischer Kohlenwasserstoff, ein Ether, ein halogenierter Kohlenwasserstoff, flüssiges Ammoniak, Methylamin oder Dimethylamin ist.

8. Verfahren nach einem vorhergehenden Anspruch, wobei das Verfahren weiter das Entfernen des Cyclopropens aus dem Reaktionsgemisch durch Destillation umfaßt.

9. Verfahren nach einem vorhergehenden Anspruch, wobei X ein Chlor- oder Bromatom ist, R ein (C₁-C₁₀) Alkylrest ist, die starke Base Natriumamid ist, die schwächere Base Hexamethyldisilazan ist und das inerte Lösungsmittel Mineralöl ist.

10. Verfahren nach Anspruch 9, wobei R eine Methylgruppe ist.

## Revendications

1. Procédé de préparation d'un cyclopropène, qui comprend la combinaison d'un composé allylique de formule 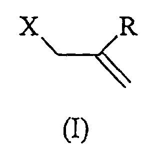 dans laquelle
X est un groupe éliminable ; et
R est un atome d'hydrogène ou un groupe alkyle, alcényle, alcynyle, cycloalkyle, cycloalkylalkyle, phényle ou naphtyle substitué ou non substitué ; où les substituants sont choisis indépendamment les uns des autres parmi les halogènes, les groupes alcoxy ou les groupes phénoxy substitués ou non substitués ;
avec une base forte non nucléophile ayant un pKa supérieur à 35, dans un solvant inerte en présence d'une quantité catalytique d'une base non nucléophile plus faible.

2. Procédé selon la revendication 1, dans lequel X est un groupe halogéno, alkyl- ou arylsulfonyloxy, alkyl- ou arylsulfate, ou alcoxy.

3. Procédé selon la revendication 2, dans lequel X est un groupe chloro, bromo, iodo, benzènesulfonyloxy, p-toluènesulfonyloxy, méthanesulfonyloxy ou tert-butoxy.

4. Procédé selon l'une quelconque des revendications précédentes, dans lequel R est un groupe alkyle en C₁-C₁₀.

5. Procédé selon l'une quelconque des revendications précédentes, dans lequel la base forte est un sel de métal alcalin d'une amine ou une base organométallique.

6. Procédé selon l'une quelconque des revendications précédentes, dans lequel la base plus faible est une silylamine ou un disilazane.

7. Procédé selon l'une quelconque des revendications précédentes, dans lequel le solvant inerte est un hydrocarbure aliphatique ou aromatique ; un éther, un hydrocarbure halogéné, de l'ammoniac liquide, une méthylamine ou une diméthylamine.

8. Procédé selon l'une quelconque des revendications précédentes, dans lequel le procédé comprend en outre l'élimination du mélange réactionnel du cyclopropène par distillation.

9. Procédé selon l'une quelconque des revendications précédentes, dans lequel X est un groupe chloro ou bromo, R est un groupe alkyle en C₁-C₁₀, la base forte est un amide de sodium, la base plus faible est un hexaméthyldisilazane, et le solvant inerte est une huile minérale.

10. Procédé selon la revendication 9, dans lequel R est un groupe méthyle.
